# EUROPEAN PATENT APPLICATION

(11) **EP 2 630 989 A1**
(43) Date of publication of application: **28.08.2013**
(21) Application number: 12156604.6
(22) Date of filing: 22.02.2012
(51) Int. Cl.: A61N 5/10

(54) **Radiotherapy apparatus**

(71) Applicant: ELEKTA AB (publ.), 103 93 Stockholm (SE)
(72) Inventor: Brown, Kevin, Horsham, Sussex RH13 5EJ (GB); Winfield, Colin, Crawley, Sussex RH11 8PU (GB); Allen, John, Haywards Heath West Sussex RH16 3EN (GB)
(74) Representative: Hall, Christopher David

(57) **Abstract**

The present invention provides a radiotherapy system and associated method which combines radiotherapy with an integrated imaging system (such as CT). Samples of imaging data are acquired at a higher frequency than the simultaneous pulses of radiation generated for therapy, and thus samples which include scattered therapeutic radiation can be discarded. The remaining samples of imaging data can be processed to provide images with reduced artefacts and increased signal-to-noise ratio.

## Description

### Technical field

The present invention relates to radiotherapy, and particularly to methods and apparatus for imaging a patient undergoing simultaneous radiotherapy treatment.

### Background

Radiotherapy is the application of high-energy ionizing radiation to treat disease and abnormal tissue in patients. Typically, the radiation is collimated into a beam and directed towards a target area for treatment. A high enough radiation dose will kill the targeted cells. However, the radiation has an adverse impact on any cells it passes through, and thus typical radiotherapy systems direct beams of radiation towards a common target (e.g. a tumour) from multiple directions. Depending on the cost and complexity associated with each radiation source, it is generally more practical to employ a single radiation source, rotating that source around the patient and thus directing the beam towards the target from multiple directions. However, systems which use low-cost radiation sources (e.g. radio-active isotopes such as cobalt-60) may employ several sources positioned around the patient and all directed towards a common target. In either case, the radiation dose in the target accumulates to a high level, while the dose in the surrounding healthy tissue is reduced.

In view of the negative impact of radiation on healthy tissue, it is a common goal in radiotherapy to ensure the radiation dose is imparted to the desired target as accurately as possible. This maximizes the therapeutic effect of the treatment, while minimizing the adverse impact on healthy tissue and reducing patient recovery times. Various means have been developed with this goal in mind.

For example, it is known to collimate the radiation beam to take a desired shape, which conforms to the cross section of the target, or to take any other shape as desired. Such collimation can be altered dynamically during rotation of the radiation source around the patient to match the changing cross-section of the target as viewed from different directions. This allows both fast and effective treatment.

In addition, the treatment itself is meticulously planned to minimize the dose delivered to healthy tissue. One example of this process is as follows. The anatomical structures in and surrounding the target are identified in a "planning scan" (i.e. a detailed image of the treatment area), and a number of clinical objectives specified. For example, a minimum dose may be specified for the target, and a maximum dose specified for critical structures. A minimum dose in this context means a radiation dose which should be exceeded as a result of the treatment; a maximum dose means a radiation dose which should not be exceeded. A treatment plan is then generated, taking into account this information as well as the geometrical constraints of the radiotherapy system in which the therapy will eventually be implemented. Treatment planning is a complicated process that requires significant computing power and resources.

However, both treatment planning and collimation require the location of the target to be accurately known at the time of treatment. The best treatment plan and the most accurate beam collimation will be compromised if the target moves from its previous position and the therapy is not updated to account for that movement. Target motion may occur over a period of time, as a result of the treatment, or instantaneously with movement of the patient (e.g. through the motion associated with respiration). It is therefore desirable to image the target area simultaneously with treatment, to allow the treatment to be updated (or in extreme circumstances, halted) in the event of movement of the target.

Various techniques have been developed. However, depending on the imaging modality employed, there are frequently difficulties in combining an imaging device within a radiotherapy system. For example, when combining a medical resonance imaging (MRI) device within a radiotherapy system, one must account for the large magnetic fields generated as part of the MRI process. These will, in general, have an effect on the radiation used to treat the patient.

Another approach has been to integrate a computed tomography (CT) system within a radiotherapy system. In such systems, a source of therapeutic radiation is placed on a gantry and, as is conventional, rotated around the patient so as to direct the therapeutic radiation towards the patient from multiple directions. In this context, the source of therapeutic radiation is usually a linear accelerator, and the energy of the therapeutic radiation is in the megavoltage (MV) range. A source of imaging radiation is mounted on the gantry at a location which is outside the main therapeutic beam. Imaging radiation is usually at a lower energy, such as the kilovoltage (kV) range, to achieve greater contrast and image quality. A detector mounted on the gantry opposite the source of imaging radiation detects the imaging radiation after it has been attenuated by the patient and generates a two-dimensional transmission image of the treatment area. A plurality of such images can be combined to reconstruct a three-dimensional CT image. The imaging radiation interacts with the patient and other objects located in its path and generates scattered radiation in all directions. Methods are well known to mitigate the effects of this low energy scatter e.g. collimation screens known as scatter grids.

A problem associated with simultaneous treatment and acquisition of CT images is that of scatter. The therapeutic beam also interacts with the patient and other objects located in its path and generates high-energy scattered radiation in all directions. A finite portion of this high-energy scattered radiation is directed towards the detector, which detects it in addition to the signal from the imaging radiation. The high-energy scattered radiation typically has two effects. One is to add a pulse artefact to the image, and the other is to add a noisy background to the image thereby reducing the signal-to-noise ratio. Conventional low-energy mitigation techniques (e.g. the scatter grids noted above) have minimal effect on this high-energy scattered radiation.

These effects can be mitigated by positioning the source and detector of imaging radiation at 90° on either side of the source of therapeutic radiation, thus reducing the percentage of scattered radiation which is directed towards the detector. However, this may have only a limited effect, and thus it is desirable to reduce the effects of scattered radiation still further.

### Summary of invention

According to a first aspect of the present invention, there is provided a radiotherapy apparatus, comprising: a source of therapeutic radiation, for generating a beam of therapeutic radiation directed towards a patient; a source of imaging radiation for generating a beam of imaging radiation directed towards the patient; a detector, arranged to detect the imaging radiation after it has passed through the patient, and to generate imaging data; and an imaging device, arranged to receive the imaging data. The beam of therapeutic radiation is pulsed at a first frequency, and the detector is arranged to acquire simultaneously samples of imaging data at a second frequency, higher than the first frequency. The samples of imaging data include first samples of imaging data corresponding to a time window in which the beam of therapeutic radiation is active and second samples of imaging data corresponding to a time window in which the beam of therapeutic radiation is inactive. The imaging device is configured to use the second samples of imaging data, and not the first samples of imaging data, to generate images of the patient.

The present invention therefore provides a radiotherapy system which combines radiotherapy with an integrated imaging system (such as CT). Samples of imaging data are acquired at a higher frequency than the simultaneous pulses of radiation generated for therapy, and thus samples which include scattered therapeutic radiation can be discarded. The remaining samples of imaging data can be processed to provide images with reduced artefacts and increased signal-to-noise ratio.

### Brief description of the drawings

For a better understanding of the present invention, and to show more clearly how it may be carried into effect, reference will now be made, by way of example, to the following drawings, in which:
Figure 1 shows a radiotherapy system according to embodiments of the present invention; and
Figure 2 is a flowchart of a method in accordance with embodiments of the present invention.

### Detailed description

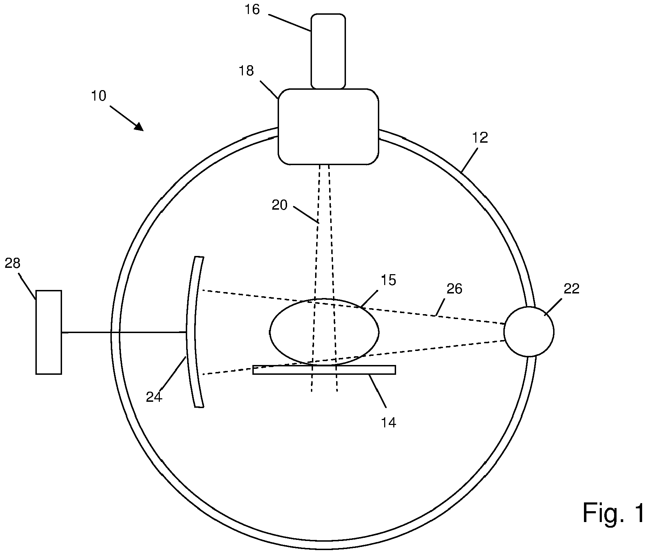

Figure 1 show a combined radiotherapy and imaging system 10 according to embodiments of the present invention.

The system comprises a rotatable gantry 12 and a patient support 14 located on or near the rotation axis of the gantry 12. In the illustrated embodiment the gantry 12 is depicted as a circular ring for simplicity, but those skilled in the art will appreciate that the gantry 12 may take any convenient form.

A source of therapeutic radiation 16 is mounted on the gantry 12 and directed inwards towards the axis of rotation. According to embodiments of the present invention, the source 16 comprises a linear accelerator, or linac, arranged to accelerate charged particles (such as electrons) to relativistic speeds and energies in the megavoltage (MV) range. In one embodiment, the charged particles are used to treat the patient directly, typically for targets on or near the surface of the patient as the particles do not penetrate deeply. In another embodiment, the particles are fired towards a high-density target (e.g. tungsten) to generate secondary radiation via mechanisms such as Bremsstrahlung radiation. The secondary radiation so generated includes x-rays up to and including the energy of the charged particle.

Linear accelerators require large amounts of power and generate extremely energetic radiation. As such it is not practical or desirable to operate them continuously. While active, therefore, a linear accelerator is pulsed periodically to generate a pulsed radiation beam. Continuous generation of radiation would require impractically large power resources, and result in catastrophic overheating of the device. In embodiments of the present invention, the linear accelerator is pulsed at a frequency in the range of 10 to 500 Hz according to the type of treatment being carried out, with each pulse having a duration of between 1 and 10 µs.

The therapeutic radiation generated by the source 16 is collimated into a beam having a primary shape (cone-shaped and fan-shaped beams are well known but other shapes are possible) by primary collimators. Further collimation is performed by secondary collimators 18, to adapt the beam to take a desired cross section. Typically the primary collimators will be fixed in place such that the overall shape of the treatment beam (i.e. before secondary collimation) is not changed during treatment. The secondary collimators tend to be more complex, however, and these may be updated during treatment to ensure the treatment beam conforms to a desired cross section. One particularly common secondary collimator is known as a multi-leaf collimator (MLC). MLCs comprise a housing defining a radiation window through which the radiation beam passes. One or more banks of parallel leaves are arranged to the side of the radiation window, with a common arrangement being to have two banks of parallel leaves arranged on opposing sides of the window. Each bank of leaves comprises a plurality of leaves arranged side-by-side in a lateral direction perpendicular to the beam axis. Each leaf is relatively narrow in that lateral direction, and relatively long in a longitudinal direction (perpendicular to both the beam axis and the lateral direction). Each leaf may be manufactured from a high-density material (such as tungsten), and has a significant depth in the direction of the beam axis in order to block the radiation from passing through. In use, the leaves are individually controllable to extend in the longitudinal direction across the radiation window to a greater or lesser extent as required. In one embodiment, each leaf can be extended across the entire radiation window or withdrawn from the entire radiation window, and can be arranged to take any position in between those two extremes. The leaves can therefore be positioned so as to define an aperture through the window of an arbitrary shape, thus collimating the radiation beam to conform to that shape.

Thus in one embodiment the collimator 18 is a multi-leaf collimator as described above. The combined effect of the source 16 and the collimator 18 is to produce a beam of radiation 20 having a collimated shape and an energy (typically in the MV range) which has a therapeutic effect in the patient. In use, the therapeutic beam 20 is directed generally towards the rotation axis of the gantry 12. A patient 15 is positioned on the support 14 such that the target for treatment lies on or near the rotation axis of the gantry 12. Rotation of the gantry 12 during treatment causes the beam 20 to be directed towards the target from multiple directions. The target remains in the treatment beam for most (or all) of the time and thus radiation dose accumulates to a relatively high level there. The surrounding healthy tissue also lies within the radiation beam 20 but only for a limited period of time before the gantry rotates and the beam passes through a different part of the patient 15. Radiation dose in the healthy tissue is therefore kept at a relatively low level.

A further source of radiation 22 is also mounted on the gantry 12. This source 22 generates radiation which is suitable for imaging (e.g. x-rays in the kV range), and provides a beam of radiation 26 directed inwards towards the rotation axis of the gantry 12. In one embodiment the beam of imaging radiation 26 has a cone shape, as suitable for use in cone beam computed tomography (CBCT), but other shapes are possible. The imaging radiation may be pulsed or continuous. In the former case, the pulse frequency may be the same as the read-out frequency of the detector 24, i.e. at least 1000 Hz and in a particular embodiment 4000 Hz (see below).

A detector 24 is mounted on the gantry 12 opposite the source of imaging radiation 22, and is arranged to capture the imaging radiation 26 after it has passed through, and been attenuated by, the patient 15. Knowledge of the attenuated radiation (i.e. the imaging data), the source radiation 26 and the attenuation mechanism allows the imaging data to be back-projected to generate a tomographic image of the patient.

In one embodiment, the detector 24 is a multi-slice detector having a plurality of rows of detector elements which allow it to reconstruct a corresponding plurality of CT slices in one rotation. The rows of detector elements are arranged parallel to one another and run in a direction orthogonal to the rotation axis of the gantry 12 (i.e. each row lies in a plane parallel to the plane of Figure 1). Each row may take a curved shape, and in one embodiment the radius of curvature is equal to the distance from the detector 24 to the source of imaging radiation 22. In one particular embodiment, the detector 24 has between 200 and 400 parallel rows of detector elements, with each row comprising between 800 and 1200 elements.

An imaging device 28 is connected to the detector 24 and processes the data sampled there to generate images of the patient 15. Further operation of the imaging device will be described below.

As described above, a problem with simultaneous imaging and therapy in systems such as that described above is that a finite percentage of the therapeutic radiation is scattered from the patient 15 into the detector 24. This percentage can be reduced by mounting the detector 24 and the source of imaging radiation 22 at positions which lie outside the therapeutic beam 20, and particularly at positions such that the imaging beam 26 is orthogonal to the therapeutic beam 20. However, a significant portion of scattered therapeutic radiation will still impinge on the detector 24. As it will typically have a higher energy than the imaging radiation, it is particularly difficult to eliminate and the imaging quality will be markedly reduced.

According to embodiments of the present invention, the therapeutic beam of radiation 20b is pulsed at a first, relatively low frequency during treatment while imaging data is sampled in the detector 24 at a second, relatively high frequency. Thus the detector 24 acquires a plurality of samples of imaging data, some acquired when both the therapeutic 20 and imaging 26 radiation beams are active and some acquired when just the imaging radiation beam 26 is active. In order to mitigate the effect of scattered therapeutic radiation on image quality, only the latter samples of imaging data are used to build an image of the patient. The former samples of data can be discarded.

This process takes place in real time and at high speed. Samples of imaging data are acquired while the source of therapeutic radiation is active, but between individual pulses of the therapeutic radiation beam.

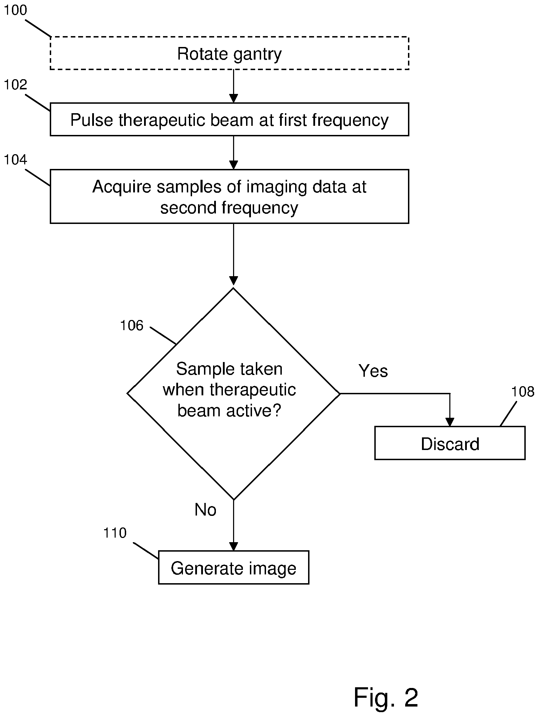

Figure 2 is a flowchart of a method according to embodiments of the present invention, as performed by the system 10 described with respect to Figure 1.

The method begins with an optional step (100) of rotating the gantry 12. In many embodiments, the gantry will be rotated during therapy and during acquisition of imaging data. Thus, therapy is delivered in one or more arcs, as the source of therapeutic radiation 16 is left active while the gantry rotates. Samples of imaging data are acquired at different angles of rotation. In one embodiment, the gantry 12 is rotated at a rotation speed in the range from 0.05 to 0.5 revolutions per second (i.e. such that one revolution is completed in between 2 and 20 seconds). In a particular embodiment, the gantry 12 is rotated at a speed of 0.2 revolutions per second (i.e. one revolution is completed in 5 seconds).

In alternative embodiments, the gantry 12 is rotated in between steps of activating the source 16 of therapeutic radiation and acquiring samples of imaging data (also known as "step and shoot"). Thus, in these embodiments samples of imaging data are again acquired at different angles of rotation, and therapy delivered from multiple angles; however, the gantry 12 is not rotated simultaneously with the therapeutic source 16 being active or imaging data being sampled.

In still further embodiments, the gantry 12 is not rotated at all and thus the gantry need not be rotatable. In these embodiments therapy is delivered from a single angle of rotation and the detector 24 also acquires samples of imaging data at a single angle of rotation. Thus only transmission images of the patient can be obtained (it is not possible to reconstruct three-dimensional images of the patient from a single angle of rotation).

In the next step (102), the therapeutic beam 20 is pulsed at a first frequency. As described above, the therapeutic beam 20 will typically have an energy in the MV range, and will have been generated using a linear accelerator. In order to avoid excessive power usage and over-heating in the linear accelerator, the beam 20 can only be generated for a fraction of the time. In embodiments of the present invention, the linear accelerator is pulsed at a frequency in the range of 10 to 500 Hz according to the type of treatment being carried out, with each pulse having a duration of between 1 and **10** µs.

The next step (104) in the flowchart takes place simultaneously with the preceding step (102) of pulsing the therapeutic beam at a first frequency. The detector 24 is controlled to acquire samples of the imaging data at a second frequency, greater than the first frequency at which the therapeutic beam is pulsed. Thus, the source of imaging radiation 22 is active, and generates a beam of imaging radiation (typically in the kV range) which is attenuated by the patient. A portion of the attenuated radiation falls on the detector 24, and charge accumulates in the various detector elements. This charge is sampled (i.e. collected) and then allowed to accumulate again. Each sample of data thus corresponds to a time window in which charge is allowed to accumulate, ending when the charge is sampled (i.e. collected). In embodiments of the present invention the second frequency is at least 1000 Hz, and in a particular embodiment the second frequency is 4000 Hz, i.e. the imaging data collected by the detector 24 is sampled every 250 µs.

The steps 102 and 104 take place simultaneously in the sense that, in a particular time window, the therapeutic beam is pulsed at a constant first frequency and the imaging data is sampled at a constant second frequency. The second frequency is greater than the first frequency, and thus some of the samples of imaging data will correspond to charge accumulated when the therapeutic beam was active, while other samples of imaging data correspond to charge accumulated when the therapeutic beam was inactive. In step 106, the imaging device 28 determines, for each sample, whether that sample corresponded to a time window in which the therapeutic beam was active or inactive. A number of different methods may be employed to achieve this. For example, the source of therapeutic radiation 16 may generate a signal, or flag, to indicate when the therapeutic beam is active. This signal can be passed to the imaging device 28. Alternatively, the imaging device may detect the presence of scattered therapeutic radiation in the sample of imaging data, for example by measuring the overall signal level for that sample. If the overall sample level is found to have increased significantly from one sample to the next, it is likely that increase is due to scattered therapeutic radiation. The overall signal level may be compared to a threshold value for the purposes of that evaluation. The threshold value may be determined based on the overall signal level for one or more previous samples.

If the sample is identified as having been acquired while the therapeutic beam was active, then it is no longer needed in the imaging process and may be discarded (step 108). If the sample is identified as having been acquired while the therapeutic beam was inactive, then it can be used as part of the process (110) to image the patient 15 undergoing therapy. The sample of imaging data may be used directly to provide a transmission image of the patient, or combined with other samples of data taken at the same angle of rotation. For example, samples taken when the therapeutic beam was inactive ("clean samples") can be sorted into one or more bins with samples of data taken at like angles of rotation included in like bins. The samples of data in each bin can be summed, integrated, averaged or otherwise combined to generate combined imaging data for a particular angle of rotation. The combined imaging data for different angles of rotation can themselves be combined to reconstruct three-dimensional images of the patient.

The images so generated can be fed back to the operator of the radiotherapy system 10, so that the target for therapy can be tracked during therapy. If the target moves unexpectedly, or if the therapeutic beam is found to be misdirected, then treatment can be updated to account for the error or halted. In some embodiments, the images generated in the imaging device 28 can be fed back to the therapeutic source 16 and the collimator 18 to automatically account for movement of the target in real time, during therapy. That is, the images can be used to control the therapeutic source 16 and/or the collimator 18.

In a typical example, the radiotherapy system 10 may be controlled with the following values:
- A rotation speed of 5 seconds per rotation (0.2 revs per second);
- First frequency of 400 Hz; and
- Second frequency of 4000 Hz.

In one five-second rotation, therefore, there will be a maximum of 2000 pulses of the therapeutic beam, and 20,000 samples of imaging data. 2000 of the samples are discarded for the purposes of generating an image, and thus the remaining 18,000 samples are processed to generate a number of average samples, e.g. 1200. These average samples can then be used for CT reconstruction, to generate a three-dimensional image of the patient.

The present invention therefore provides a radiotherapy system and associated method which combines radiotherapy with an integrated imaging system (such as CT). Samples of imaging data are acquired at a higher frequency than the simultaneous pulses of radiation generated for therapy, and thus samples which include scattered therapeutic radiation can be discarded. The remaining samples of imaging data can be processed to provide images with reduced artefacts and increased signal-to-noise ratio.

Those skilled in the art will appreciate that various amendments and alterations can be made to the embodiments described above without departing from the scope of the invention as defined in the claims appended hereto.

## Claims

1. A radiotherapy apparatus, comprising:
a source of therapeutic radiation, for generating a beam of therapeutic radiation directed towards a patient;
a source of imaging radiation for generating a beam of imaging radiation directed towards the patient;
a detector, arranged to detect the imaging radiation after it has passed through the patient, and to generate imaging data; and
an imaging device, arranged to receive the imaging data;
wherein the beam of therapeutic radiation is pulsed at a first frequency;
wherein the detector is arranged to acquire simultaneously samples of imaging data at a second frequency, higher than the first frequency, the samples of imaging data including first samples of imaging data corresponding to a time window in which the beam of therapeutic radiation is active and second samples of imaging data corresponding to a time window in which the beam of therapeutic radiation is inactive; and
wherein the imaging device is configured to use the second samples of imaging data, and not the first samples of imaging data, to generate images of the patient.

2. The radiotherapy apparatus according to claim 1, wherein the source of therapeutic radiation, the source of imaging radiation and the detector are each rotatable around a common axis of rotation.

3. The radiotherapy apparatus according to claim 2, wherein, in use, the patient is placed on or near the axis of rotation.

4. The radiotherapy apparatus according to claim 2 or 3, wherein the imaging device is configured to reconstruct a three-dimensional image of the patient from said second samples of imaging data.

5. The radiotherapy apparatus according to any one of claims 2 to 4, wherein the source of therapeutic radiation, the source of imaging radiation and the detector are each configured to rotate around the axis of rotation at a rotation speed in the range from 0.05 to 0.5 revolutions per second.

6. The radiotherapy apparatus according to any one of the preceding claims, wherein the first frequency is in the range 10 to 500 Hz.

7. The radiotherapy apparatus according to any one of the preceding claims, wherein each pulse of the beam of therapeutic radiation has a duration in the range from 1 to 10 µs.

8. The radiotherapy apparatus according to any one of the preceding claims, wherein the second frequency is at least 1000 Hz.

9. The radiotherapy apparatus according to any one of the preceding claims, wherein the imaging device is configured to determine which samples are first samples, and which samples are second samples.

10. The radiotherapy apparatus according to claim 9, wherein the imaging device is configured to receive a signal indicating when the beam of therapeutic radiation is active.

11. The radiotherapy apparatus according to claim 9, wherein the imaging device is configured to determine an overall signal level for each sample of imaging data, and wherein the sample is identified as a first sample if the overall signal level exceeds a threshold.

12. The radiotherapy apparatus according to any one of the preceding claims, wherein the imaging device is configured to integrate the second samples of imaging data.
